# DEMANDE DE BREVET EUROPEEN

(11) **EP 0 925 804 A1**
(43) Date de publication de la demande: **30.06.1999**
(21) Numéro de dépôt: 98403039.5
(22) Date de dépôt: 04.12.1998
(51) Int. Cl.: A61M 27/00

(54) **Dispositif de drainage externe de fluide biologique**

(30) Priorité: 17.12.1997 FR 9716020; 10.07.1998 FR 9808871
(71) Demandeur: NMT Neurosciences Implants S.A., 06921 Sophia Antipolis (FR)
(72) Inventeur: Lecuyer, Alain, 06130 Grasse (FR); Amann, Bernard, St. Roman de Bellot, 06200 Nice (FR); Rolland, Didier, 06560 Valbonne (FR)
(74) Mandataire: Geismar, Thierry

(57) **Abrégé**

L'invention concerne un dispositif de drainage externe de fluide biologique, notamment de liquide céphalo-rachidien, comprenant un cathéter (1,2) dont une extrémité est disposée dans la zone à drainer et l'autre extrémité est reliée à un sac de drainage (3).

Ce dispositif comprend des moyens de mesure de pression (4) pour mesurer la pression dans la zone à drainer et des moyens de commande (5) de débit extérieurs sur ledit cathéter. Une alimentation (10) à commande électronique est prévue pour actionner lesdits moyens de commande de débit lorsque la pression dépasse un seuil prédéterminé.

## Description

La présente invention concerne un dispositif de drainage externe de fluide biologique, et notamment de liquide céphalo-rachidien.

On se référera plus particulièrement dans ce qui suit à des dispositifs de drainage ventriculaires destinés à la régulation de la pression intracrânienne. Toutefois, le site de drainage peut aussi bien être l'espace lombaire, l'espace sous-arachnoïdien ou tout autre site permettant le drainage, notamment de liquide céphalo-rachidien

On connaît déjà divers types de valves implantables destinées à contrôler le drainage d'un excès de liquide céphalo-rachidien contenu dans les ventricules cérébraux vers, par exemple, la cavité péritonéale. Ces valves sont généralement destinées au traitement de l'hydrocéphalie.

C'est ainsi qu'il est connu d'utiliser de simples clapets régulateurs de pression. Ces valves présentent l'inconvénient de pas tenir compte des différences de hauteur d'eau en fonction de la position, notamment couchée ou debout, du patient.

On a également proposé des valves à pointeau et membrane présentant une caractéristique dite "anti-siphon", et évitant donc l'inconvénient ci-dessus. Bien que donnant souvent satisfaction, elles sont destinées à être implantées, et ne conviennent donc pas pour un usage de courte durée, par exemple à la suite d'un traumatisme.

Quant aux systèmes de drainage externe connus, ils sont constitués d'un simple cathéter dont une extrémité est disposée dans la zone à drainer et l'autre extrémité est reliée à un sac de drainage. Ne disposant pas de moyens de régulation de pression ayant une caractéristique du type "anti-siphon", ils ne permettent pas au patient de se tenir debout. Ils nécessitent d'autre part des vérifications de réglage après des mouvements du patient (toilette, transport,...).

La simple addition d'un régulateur de pression ne constitue pas une solution convenable. Ces régulateurs sont en effet des dispositifs relativement compliqués sujets à des obstructions dans le cas où le liquide céphalo-rachidien est mêlé à du sang. Leur coût est en outre élevé.

La présente invention vise à pallier ces inconvénients.

A cet effet, l'invention a pour objet un dispositif de drainage externe de fluide biologique, notamment de liquide céphalo-rachidien, comprenant un cathéter dont une extrémité est disposée dans la zone à drainer et l'autre extrémité est reliée à un sac de drainage, caractérisé par le fait qu'il comprend des moyens de mesure de pression pour mesurer la pression dans la zone à drainer et des moyens de commande de débit extérieurs sur ledit cathéter, une alimentation à commande électronique étant prévue pour actionner lesdits moyens de commande de débit lorsque la pression dépasse un seuil prédéterminé.

On régule donc ici directement et simplement la pression dans la zone à drainer. Les moyens de commande de débit et leur alimentation sont extérieurs au circuit de fluide céphalo-rachidien et sont donc réutilisables. Seuls sont jetables le cathéter, le sac de drainage, et éventuellement les moyens de mesure de pression si le fluide à drainer a directement accès à ces derniers.

Dans le cas du drainage ventriculaire externe, un tel dispositif est insensible à la position du patient qui peut se lever et se déplacer, ou être bougé sans réglage du système.

Divers types de moyens de commande de débit peuvent être utilisés.

Dans un premier cas, il peut s'agir de moyens progressifs tels qu'une pompe ou un robinet pilotés par un moteur.

Dans un autre cas, il peut s'agir de moyens fonctionnant en mode toutou-rien tels qu'une valve à pincement ou un clapet.

Dans ce dernier cas, la valve est normalement fermée et s'ouvre lorsque le seuil de pression est atteint. Cette ouverture peut être par impulsions, les impulsions se succédant tant que la pression n'est pas revenue en dessous du seuil.

Dans un mode de réalisation particulier, les moyens de mesure de pression sont disposés sur la ligne de drainage. Plus particulièrement, ils peuvent être disposés entre une partie de cathéter dont une extrémité est introduite dans le ventricule à drainer et une autre partie de cathéter reliée au sac de drainage.

Toutefois, dans un autre mode de réalisation, on peut utiliser un cathéter implantable indépendant comportant un capteur dans sa partie distale. Dans le cas du drainage ventriculaire, on peut utiliser tout autre moyen de mesure de pression intracrânienne délivrant un signal compatible avec la commande électronique de l'alimentation.

Egalement dans un mode de réalisation particulier, la commande électronique est agencée pour relever la pression à des intervalles de temps prédéterminés et, si la pression est supérieure au seuil, pour actionner lesdits moyens de commande de débit pendant une durée également prédéterminée.

On décrira maintenant, à titre d'exemple non limitatif, un mode de réalisation particulier de l'invention, en référence aux dessins schématiques annexés dans lesquels:
- la figure 1 représente les différents éléments d'un dispositif selon l'invention pour le drainage ventriculaire externe, tels que mis en place sur un patient;
- la figure 2 est une vue en coupe de la valve de ce dispositif; et
- la figure 3 illustre le principe de la régulation.

Le dispositif selon l'invention comprend un cathéter de drainage en deux parties, l'une 1 du côté de la tête du patient et l'autre 2 du côté d'un sac de drainage 3. Un capteur de pression 4 de tout type connu est disposé entre les parties 1 et 2 du cathéter. Ce capteur est disposé à proximité immédiate de la tête du patient pour mesurer la pression intracrânienne indépendamment de la position du patient.

Une des extrémités de la partie 1 du cathéter est introduite de façon connue dans un ventricule à drainer du cerveau du patient. Son autre extrémité est connectée à l'entrée du capteur de pression 4.

La sortie du capteur 4 est reliée à une extrémité de la partie 2 du cathéter de drainage. L'autre extrémité de cette partie 2 est connectée à un sac de drainage 3. Cette ligne de drainage pourrait d'ailleurs être elle-même réalisée en plusieurs parties

Le sac 3 est réalisé en matière plastique transparente et est gradué pour permettre de mesurer la quantité de fluide drainée. Il peut également être muni d'un système compte-gouttes, par exemple optique.

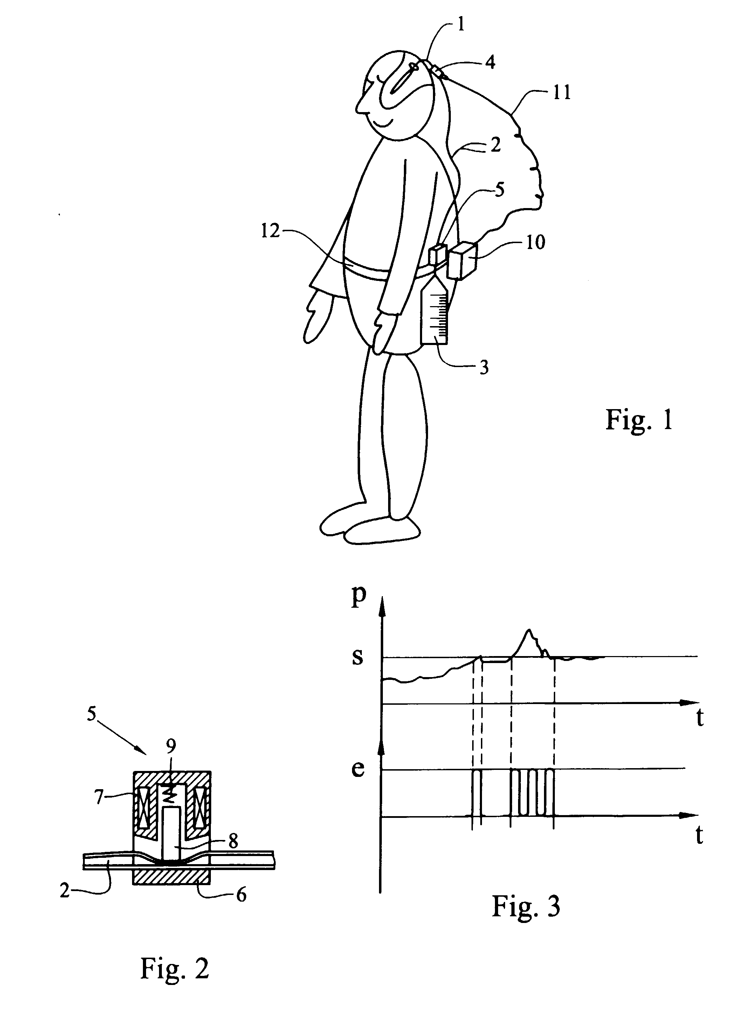

Une valve à pincement 5 est disposée sur la partie 2 du cathéter de drainage. Cette valve est composée d'un corps 6 formant un étrier dans lequel le cathéter est engagé pour être fermé par pincement, comme montré à la figure 2 qui représente la valve à plus grande échelle.

Une des branches du corps 6 reçoit un électroaimant dont le bobinage 7 est alimenté comme cela sera décrit ci-après. Le noyau 8 forme un plongeur susceptible de pincer le cathéter 2 contre l'autre branche du corps 6. Un ressort de compression 9 est disposé entre le corps 6 et le noyau 8 pour pousser le noyau en direction du cathéter 2 et le pincer.

Un boîtier électronique 10 d'alimentation reçoit du capteur 4 par un fil 11 un signal électrique représentatif de la pression *p* mesurée et le compare à une valeur de seuil *s* pour commander l'ouverture de la valve lorsque la pression dépasse ce seuil. Par ailleurs le boîtier 10 assure l'alimentation électrique du capteur 4.

La figure 3 montre un exemple d'évolution dans le temps de la pression intracrânienne mesurée *p* et de la tension *e* de commande de l'électroaimant qui en résulte.

En absence d'alimentation de l'électroaimant, la valve 5 est normalement fermée. Lorsque la pression *p* dépasse le seuil *s*, l'électronique du boîtier 10 est agencée pour envoyer dans le bobinage 7 des impulsions de tension de manière à rétracter le noyau 8 contre l'action du ressort 9 et ainsi ouvrir la valve. Du fluide s'écoule et la pression baisse. Lorsqu'elle devient inférieure au seuil, la valve n'est plus commandée.

On observera que l'on peut réaliser un lissage du signal issu du capteur 4.

On peut également ne relever la pression qu'à des intervalles de temps prédéterminés, par exemple toutes les 10 secondes et, si la pression est supérieure au seuil, maintenir la valve ouverte pendant une durée également prédéterminée, par exemple pendant une seconde. On peut ainsi obtenir une limitation du débit.

Enfin, le dispositif est avantageusement complété par une ceinture 12 à laquelle peuvent être fixés le boîtier 10 d'alimentation et le sac 3 de drainage. Le patient est ainsi parfaitement mobile.

Bien entendu, le boîtier pourrait être fixé au lit du patient ou à un support proche du lit.

L'électronique qui a été décrite est réduite à sa plus simple expression. Mais on pourrait aussi prévoir des moyens de raccordement à un enregistreur ou à un micro-ordinateur pour enregistrer, notamment, l'évolution de la pression en fonction du temps et procéder simultanément à son analyse. En variante, des moyens de mémorisation pourraient être prévus dans le boîtier électronique, les enregistrements pouvant être analysés a posteriori sur un micro-ordinateur.

Il est également possible d'utiliser des moyens de mesure de pression reliés à un centre de surveillance général d'un établissement hospitalier, chargé notamment de générer des alarmes en cas de nécessité. Dans ce cas, la valve à pincement, ou tout autre dispositif de régulation, pourrait elle-même être pilotée à distance à partir de ce centre de surveillance.

Le boîtier pourrait comporter lui-même des alarmes et/ou des affichages.

## Revendications

1. Dispositif de drainage externe de fluide biologique, notamment de liquide céphalo-rachidien, comprenant un cathéter (1,2) dont une extrémité est disposée dans la zone à drainer et l'autre extrémité est reliée à un sac de drainage (3), caractérisé par le fait qu'il comprend des moyens de mesure de pression (4) pour mesurer la pression dans la zone à drainer et des moyens de commande (5) de débit extérieurs sur ledit cathéter, une alimentation (10) à commande électronique étant prévue pour actionner lesdits moyens de commande de débit lorsque la pression dépasse un seuil prédéterminé.

2. Dispositif selon la revendication 1, dans lequel lesdits moyens de commande de débit sont des moyens progressifs.

3. Dispositif selon la revendication 1, dans lequel lesdits moyens de commande de débit comprennent une valve à pincement.

4. Dispositif selon l'une quelconque des revendications 1 à 3, dans lequel lesdits moyens de mesure de pression sont disposés sur la ligne de drainage.

5. Dispositif selon la revendication 4, dans lequel lesdits moyens de mesure de pression sont disposés entre une partie (1) de cathéter dont une extrémité est introduite dans la zone à drainer et une autre partie (2) de cathéter reliée au sac de drainage.

6. Dispositif selon l'une quelconque des revendications 1 à 3, dans lequel lesdits moyens de mesure de pression comprennent un cathéter implantable indépendant comportant un capteur dans sa partie distale.

7. Dispositif selon l'une quelconque des revendications 1 à 6, dans lequel la commande électronique est agencée pour relever la pression à des intervalles de temps prédéterminés et, si la pression est supérieure au seuil, pour actionner lesdits moyens de commande de débit pendant une durée également prédéterminée.
